(19) Europäisches Patentamt
European Patent Office
Office européen des brevets

(11) EP 0 660 701 B1

(12) # FASCICULE DE BREVET EUROPEEN

(45) Date de publication et mention de la délivrance du brevet: **11.06.1997 Bulletin 1997/24**

(21) Numéro de dépôt: **93920883.1**

(22) Date de dépôt: **15.09.1993**

(51) Int. Cl.[6]: **A61K 7/42**, A61K 7/48, A61K 7/06

(86) Numéro de dépôt international: **PCT/FR93/00886**

(87) Numéro de publication internationale: **WO 94/06404 (31.03.1994 Gazette 1994/08)**

(54) **COMPOSITION COSMETIQUE FILTRANTE PHOTOSTABLE CONTENANT UN FILTRE UV-A ET UN POLYMERE FILTRE DU TYPE SILICONE BENZOTRIAZOLE**

PHOTOSTABILE, FILTRIERENDE, KOSMETISCHE ZUSAMMENSETZUNG ENTHALTEND EINEN UVA-FILTER UND EIN BENZOTRIAZOL-SILIKON-POLYMER

PHOTOSTABLE FILTERING COSMETIC COMPOSITION CONTAINING A UV-A FILTER AND A FILTERING POLYMER OF THE BENZOTRIAZOLE SILICONE TYPE

(84) Etats contractants désignés:
**AT BE CH DE DK ES FR GB GR IE IT LI LU MC NL PT SE**

(30) Priorité: **17.09.1992 FR 9211099**

(43) Date de publication de la demande: **05.07.1995 Bulletin 1995/27**

(73) Titulaire: **L'OREAL**
**75008 Paris (FR)**

(72) Inventeurs:
- **HANSENNE, Isabelle**
  **F-75017 Paris (FR)**
- **FORESTIER, Serge**
  **F-77410 Claye-Souilly (FR)**
- **DEFLANDRE, André**
  **F-60560 Orry-la-Ville (FR)**

(74) Mandataire: **Casalonga, Axel et al**
**BUREAU D.A. CASALONGA - JOSSE**
**Morassistrasse 8**
**80469 München (DE)**

(56) Documents cités:
**EP-A- 0 365 370**
**EP-A- 0 392 883**
**FR-A- 2 440 933**
**FR-A- 2 680 683**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 660 701 B1

Printed by Rank Xerox (UK) Business Services
2.14.7/3.4

## Description

La présente invention est relative à une composition cosmétique photostable destinée à protéger la peau et les cheveux du rayonnement UV, contenant un filtre UV-A et un polymère filtre du type silicone benzotriazole, à son utilisation pour la protection de la peau et des cheveux contre les rayons UV et à un procédé de stabilisation du filtre UV-A par une silicone benzotriazole.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques. Il est donc souhaitable de filtrer aussi le rayonnement UV-A.

Le brevet français n° 2 440 933 décrit le 4-(tert.-butyl) 4'-méthoxy dibenzoylméthane à titre de filtre UV-A. Il est proposé d'associer ce filtre UV-A particulier, vendu sous la dénomination "PARSOL 1789"® par la Société GIVAUDAN, à différents filtres UV-B dans le but d'absorber l'ensemble des rayons UV de longueurs d'onde comprises entre 280 et 380 nm.

Malheureusement, lorsque ce filtre UV-A est utilisé seul ou en association avec des filtres UV-B, il ne possède pas une stabilité photochimique satisfaisante pour garantir une protection constante de la peau durant une exposition solaire prolongée, ce qui nécessite des applications répétées à intervalles réguliers et rapprochés si l'on veut obtenir une protection efficace de la peau contre les rayons UV.

La demanderesse a découvert qu'en associant, dans des proportions et dans un rapport en poids définis, le 4-(tert.-butyl)4'-méthoxydibenzoylméthane à un polymère filtre du type silicone benzotriazole comportant au moins une unité de formule :

$$X - \overset{R'_a}{\overset{|}{Si}} - O_{\frac{3-a}{2}} \qquad (I)$$

dans laquelle

R' désigne un groupe hydrocarboné saturé ou insaturé en $C_1$-$C_{30}$, un groupe hydrocarboné halogéné en $C_1$-$C_8$ ou un groupe triméthylsilyloxy;
a = 1 ou 2;
X = - A - Y où A représente un radical divalent hydrocarboné aliphatique ou aromatique comportant au moins 2 atomes de carbone et renfermant éventuellement un ou plusieurs atomes d'oxygène;
Y représente un reste 2-(2'-hydroxyphényl)benzotriazole portant éventuellement, sur un ou les deux noyaux aromatiques, un ou plusieurs substituants alkyle en $C_1$-$C_8$, alcényle en $C_2$-$C_8$, halogène, alcoxy, carboxy, hydroxy ou amino, on obtenait d'une manière surprenante, une stabilité photochimique satisfaisante du 4-(tert.-butyl) 4'-méthoxydibenzoylméthane. On dispose ainsi d'une association stabilisée protégeant l'épiderme humain et les cheveux contre les rayons UV de longueurs d'onde comprises entre 280 et 380 nm.

De plus, une telle association confère à la composition filtrante la contenant, outre un bon indice de protection dans l'UV-A et un bon facteur de protection solaire, des propriétés cosmétiques améliorées au niveau de l'effet collant à l'application et de l'aspect gras en final après pénétration, ainsi qu'une bonne rémanence à l'eau, c'est-à-dire une bonne stabilité de l'indice de protection au cours du temps, notamment après la douche ou la baignade.

L'indice de protection dans l'UV-A et le facteur de protection solaire (SPF) sont déterminés en utilisant la méthode in vitro décrite par B.L. DIFFEY et al. dans J.Soc. Cosmet. Chem. 40-127-133 (1989).

Cette méthode consiste à déterminer les facteurs de protection monochromatique tous les 5 nm dans une gamme de longueurs d'onde de 290 à 400 nm et à calculer à partir de ceux-ci le facteur de protection solaire.

En plus des unités de formule (I), le polymère filtre peut comporter des unités de formule :

$$R'_b - Si\,O_{\frac{4-b}{2}} \qquad (II)$$

dans laquelle R' a la même signification que dans la formule (I);

b est un nombre entier désignant 1, 2 ou 3.

A titre de groupe hydrocarboné, on peut citer les radicaux alkyle en $C_1$-$C_{30}$, alcényle en $C_2$-$C_{30}$, cycloalkyle ou aromatique comme phényle ou tolyle.

A titre de groupe hydrocarboné halogéné, on peut citer le radical 3,3,3,-trifluoropropyle.

Dans le polymère filtre constitué de motifs (I) et éventuellement (II), au moins 40% en nombre des radicaux R' sont des radicaux méthyle. Le nombre total des unités (I) et (II) est de préférence inférieur ou égal à 250 et est compris en particulier entre 2 et 50.

De tels polymères filtres à chaîne siloxanique sont décrits notamment dans les demandes de brevet européen n° 0 388 218 et 0 392 883.

De par leur caractère lipophile, les filtres utilisés se répartissent uniformément dans les supports cosmétiques classiques contenant au moins une phase grasse ou se présentant sous forme de dispersions aqueuses de vésicules lipidiques et peuvent ainsi être appliqués sur la peau pour constituer un film protecteur efficace.

La présente invention a ainsi pour objet une composition cosmétique photostable, protégeant la peau ou les cheveux contre le rayonnement UV de longueurs d'onde comprises entre 280 et 380 nm, et comprenant, dans un support cosmétiquement acceptable contenant au moins une phase grasse, 0,5 à 4% en poids de 4-(tert.-butyl) 4'-méthoxydibenzoylméthane et 0,1 à 20% en poids, de préférence 0,5 à 15%, de silicone benzotriazole telle que définie ci-dessus, le rapport en poids de la silicone benzotriazole au 4-(tert.-butyl) 4'-méthoxydibenzoylméthane étant compris entre 1 et 10.

La limite supérieure de ce rapport est déterminée par la solubilité des filtres dans la phase grasse utilisée dans la composition ou dans la phase lipidique présente dans la dispersion vésiculaire.

La présente invention a aussi pour objet un procédé de traitement cosmétique de la peau ou des cheveux destiné à les protéger contre les effets des rayons UV de longueurs d'onde comprises entre 280 et 380 nm, consistant à appliquer sur ceux-ci une quantité efficace d'une composition cosmétique telle que définie ci-dessus.

Un autre objet de la présente invention est constitué par un procédé de stabilisation du 4-(tert.-butyl) 4'-méthoxy dibenzoylméthane vis-à-vis du rayonnement UV à l'aide d'une silicone benzotriazole telle que définie ci-dessus, procédé dans lequel on utilise 0,1 à 20% en poids de silicone benzotriazole définie ci-dessus pour stabiliser de 0,5 à 4% en poids de 4-(tert.-butyl) 4'-méthoxydibenzoylméthane, le rapport en poids de la silicone benzotriazole au 4-(tert.-butyl) 4'-méthoxydibenzoylméthane étant compris entre 1 et 10.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, d'huile, de dispersion vésiculaire, d'émulsion telle qu'une crème ou un lait, de poudre, de bâtonnet solide et éventuellement être conditionnée en aérosol et se présenter sous forme de mousse ou de spray.

Elle peut contenir les adjuvants cosmétiques habituellement utilisés tels que des corps gras, des solvants organiques, des silicones, des épaississants, des adoucissants, des agents anti-mousse, des agents hydratants, des parfums, des conservateurs, des tensio-actifs anioniques, cationiques, non-ioniques, amphotères ou leurs mélanges, des charges, des séquestrants, des polymères anioniques, cationiques, non-ioniques, amphotères, ou leurs mélanges, des propulseurs, des agents alcalinisants ou acidifiants, des colorants, des pigments d'oxydes métalliques de granulométrie comprise entre 100 nm et 20 000 nm comme les oxydes de fer, ou tout autre ingrédient habituellement utilisé en cosmétique.

Les corps gras peuvent être constitués par une huile ou une cire ou leur mélange, des acides gras, des esters d'acides gras, des alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin, les huiles de silicone et les isoparaffines.

Les cires sont choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

La composition selon l'invention peut également contenir des nanopigments d'oxyde métallique dispersés dans la phase grasse et/ou dans la phase aqueuse.

Elle peut aussi contenir d'autres filtres UV lipophiles, et notamment UV-B.

La composition selon l'invention peut se présenter sous forme de dispersion vésiculaire de lipides amphiphiles ioniques ou non-ioniques, préparée selon des procédés connus. On peut, par exemple, faire gonfler les lipides dans une solution aqueuse pour former des sphérules dispersées dans le milieu aqueux comme décrit dans l'article BANGHAM, STANDISH & WATKINS, J. mol. Biol., 13, 238 (1965) ou dans les brevets FR-2 315 991 et 2 416 008 de la demanderesse. On trouvera la description des divers modes de préparation dans "Les liposomes en biologie cellulaire et pharmacologie". Edition INSERM/John Libbery Eurotext, 1987, pages 6 à 18.

Lorsque la composition se présente sous forme d'émulsion ou de dispersion vésiculaire, la phase aqueuse peut contenir des filtres UV hydrosolubles tels que l'acide benzène 1,4-[di(3-méthylidène 10-camphosulfonique)], l'acide 2-phénylbenzimidazole 5-sulfonique ou l'acide 2-hydroxy 4-méthoxybenzophénone 5-sulfonique, ces acides étant salifiés ou non.

Dans le cas d'une composition conditionnée en aérosol, on utilise des propulseurs classiques tels que les alcanes, les fluoroalcanes et les chlorofluoroalcanes.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire, d'huile ou encore sous forme d'émulsion telle qu'une crème ou un lait, sous forme de pommade, de gel, de bâtonnet solide ou de mousse aérosol.

Lorsque la composition cosmétique selon l'invention est utilisée pour la protection des cheveux, elle peut se présenter sous forme de shampooing, de lotion, de gel, d'émulsion, de dispersion vésiculaire, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encre appelé "eye liner", elle peut se présenter sous forme solide ou pâteuse, anhydre ou aqueuse, comme des émulsions huile-dans-eau ou eau-dans-huile, des dispersions vésiculaires ou encore des suspensions.

L'invention sera mieux illustrée par les exemples ci-après.

**EXEMPLE 1**

On prépare une émulsion anti-solaire huile-dans-eau de composition suivante :

- 4-tert.-butyl 4'-méthoxydibenzoylméthane 2 g
- Polydiméthylsiloxane à greffons 2 -(3'-triméthylène-2'-hydroxy-5'-méthylphényl) benzotriazole correspondant à la demande de brevet EP O 392 883, de formule 6 g

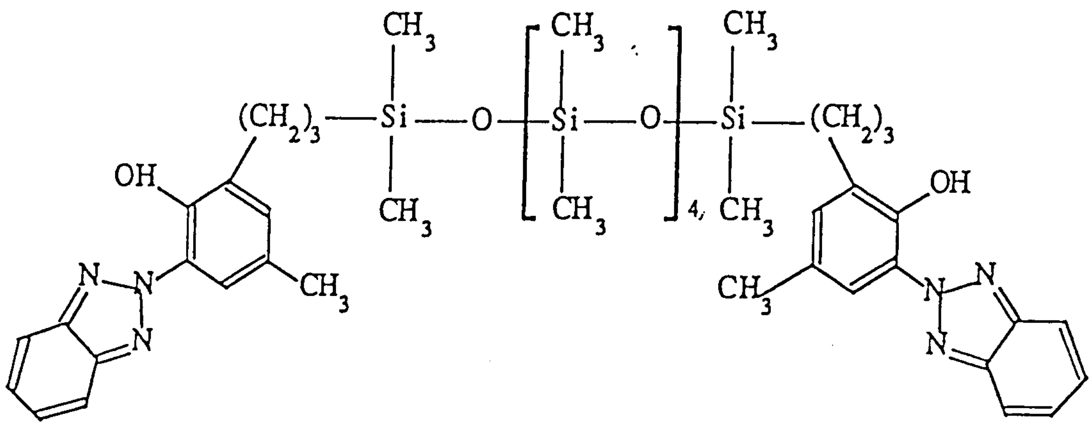

- Mélange d'alcool cétylstéarylique et d'alcool cétylstéarylique oxyéthyléné à 33 moles d'oxyde d'éthylène vendu sous la dénomination "SINNOWAX AO"® par la Société HENKEL 7 g
- Mélange de mono- et distéarate de glycol non autoémulsionnable 2 g
- Alcool cétylique 1,5 g
- Polydiméthylsiloxane vendu sous la dénomination DC 200-350 CST® par la Société DOW CORNING 1,5 g
- Triglycérides d'acides caprique et caprylique vendus sous la dénomination MIGLYOL 812® par la Société HULS 15 g
- Glycérine 20 g

- conservateurs qs.
- Agent séquestrant 0,1 g
- Eau qsp 100 g

**EXEMPLE 2**

On prépare une huile antisolaire de composition suivante :

- 4-tert.-butyl 4'-méthoxydibenzoylméthane 3 g
- Polydiméthylsiloxane à greffon 2-(3'-triméthylène-2'-hydroxy-5'-méthylphényl) benzotriazole de l'exemple 1 de la demande EP-A-0 392 883, de formule : 8 g

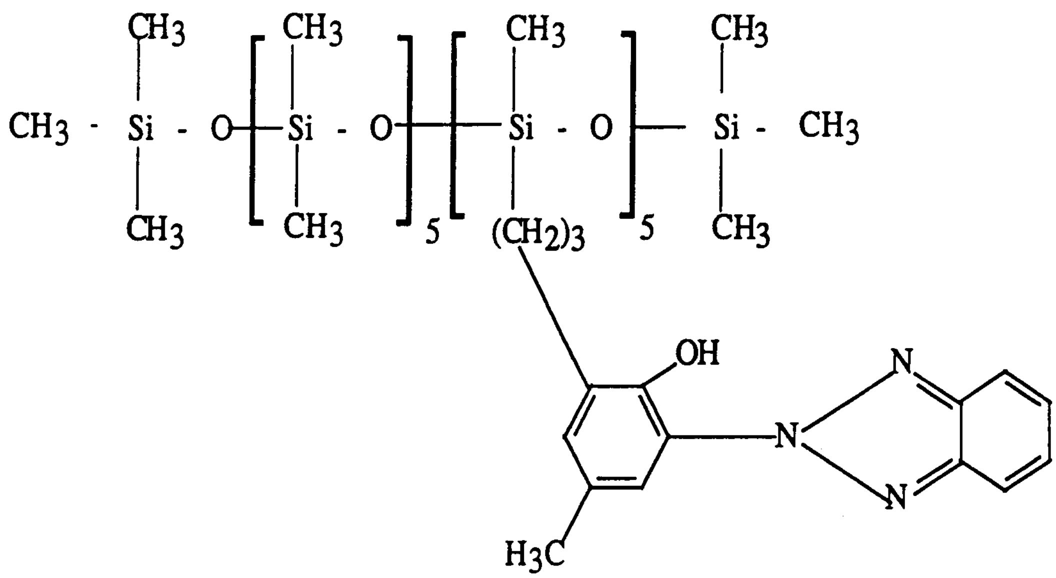

-Nanopigment d'oxyde de titane vendu sous la dénomination $TiO_2$ MOTG par la Société TIOXIDE 5 g MA

- adipate de diisopropyle qsp 100 g

**EXEMPLE 3**

On prépare une émulsion antisolaire de composition suivante :

- 4-tert.-butyl 4'-méthoxydibenzoylméthane 1 g

- Polydiméthylsiloxane à greffon 2-(3'-triméthylène-2'-hydroxy-5'-méthylphényl) benzotriazole de l'exemple 1 de la demande EP-A-0 388 218, de formule : 5 g

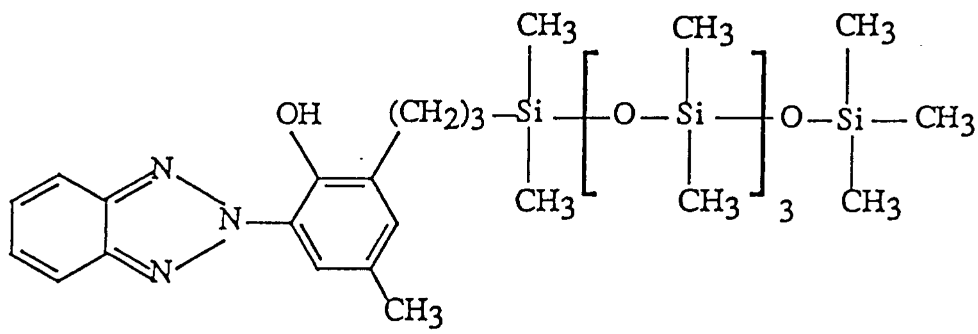

| | | | |
|---|---|---|---|
| -Monostéarate de sorbitane | | | 5 g |
| -Silice pyrogénée hydrophobe vendue sous la dénomination AEROSIL R 972® par la Société DEGUSSA | | | 0,5 g |
| - Acide benzène 1,4-di(3-méthylidène-10-campho-sulphonique) | | | 3 g |
| - Triéthanolamine | qs | pH 7 | |
| -Benzoate d'alcools en $C_{12}$-$C_{15}$ vendu sous la dénomination "FINSOLV TN"® par la Société WITCO | | | 15 g |
| - Eau purifiée | | qsp | 100 g |

**EXEMPLE 4**

On prépare une crème de jour pour le visage de composition suivante :

| | | |
|---|---|---|
| - 4-tert.-butyl 4'-méthoxydibenzoylméthane | | 1,5 g |
| - Polydiméthylsiloxane à greffons 2-(3'-triméthylène-2'-hydroxy-5'-méthylphényl) benzotriazole correspondant à la demande de brevet EP O 392 883, de formule | | 1,5 g |

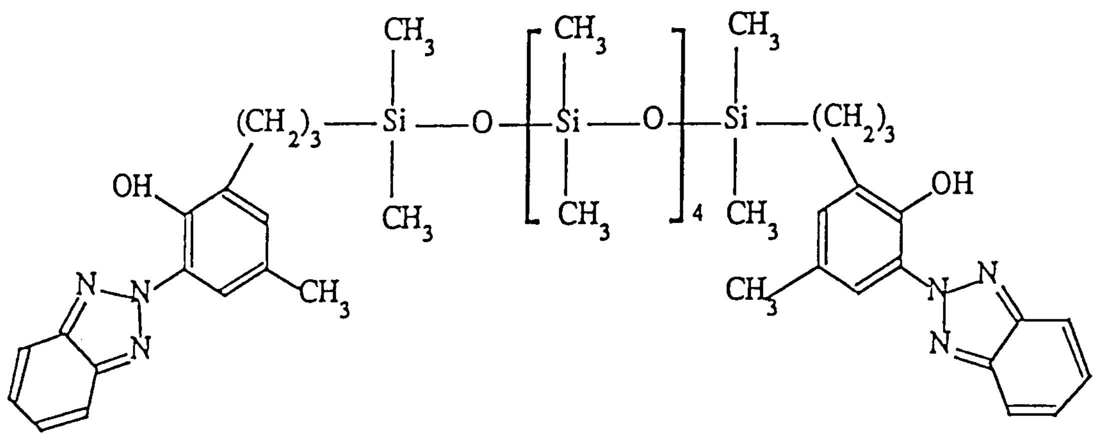

| | | |
|---|---|---|
| - Mélange d'alcool cétylstéarylique et d'alcool cétylstéarylique oxyéthyléné à 33 moles d'oxyde d'éthylène vendu sous la dénomination "SINNOWAX AO"® par la Société HENKEL | | 7 g |
| - Mélange de mono- et distéarate de glycol non autoémulsionnable | | 2 g |
| - Alcool cétylique | | 1,5 g |
| - Polydiméthylsiloxane vendu sous la dénomination DC 200-350 CST® par la Société DOW CORNING | | 1,5 g |
| -Triglycérides d'acides caprique et caprylique vendu sous la dénomination MIGLYOL 812® par la Société HULS | | 15 g |
| - Glycérine | | 20 g |
| - conservateurs qs. | | |
| -Agent séquestrant | | 0,1 g |
| - Eau | qsp | 100 g |

**EXEMPLE 5**

**Lotion de protection capillaire** :

| | |
|---|---|
| - 4-tert.-butyl 4'-méthoxydibenzoylméthane | 0,5 g |
| - Silicone à greffons 2 -(3'-triméthylène-2'-hydroxy-5'-méthylphényl) benzotriazole de formule (silicone B) : | 2 g |

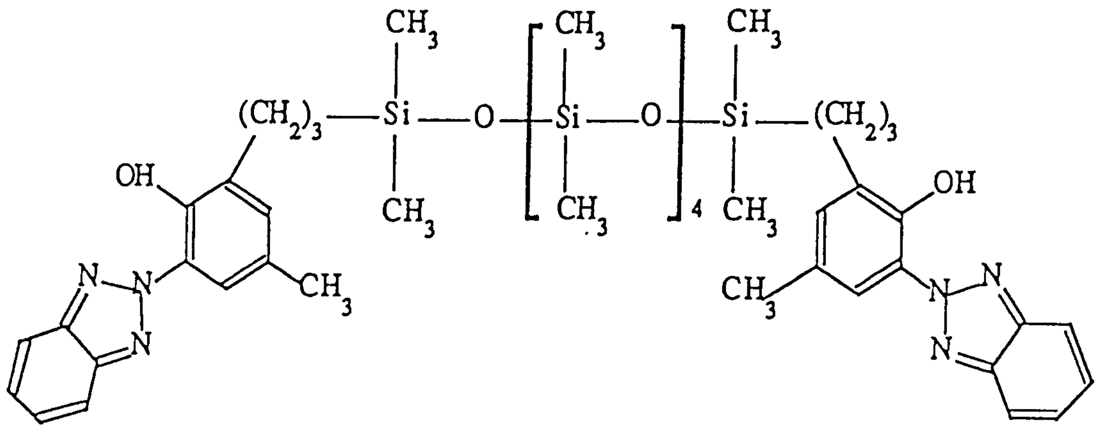

| | | |
|---|---|---|
| - Benzoate d'alcools en $C_{12}/C_{15}$, vendu sous la dénomination "FINSOLV TN"® par la Société WITCO | | 12 g |
| - Alcool éthylique | qsp | 100 g |

**EXEMPLE 6**

**Emulsion anti-solaire huile-dans-eau** :

| | | |
|---|---|---|
| - 4-tert.-butyl 4'-méthoxydibenzoylméthane | | 1,75 g |
| - Silicone à greffon 2 -[3'-(2"-méthyltriméthylène)-2'-hydroxy-5'-méthylphényl]benzotriazole de formule (silicone C) : | | 5 g |

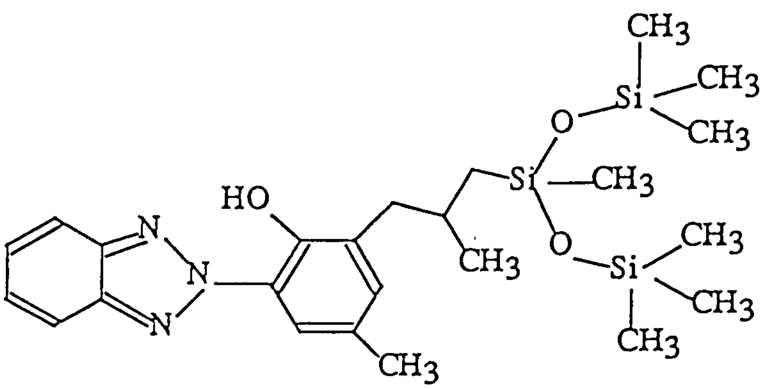

| | | |
|---|---|---|
| - Mélange d'alcool cétylstéarylique et d'alcool cétylstéarylique oxyéthyléné à 33 moles d'oxyde d'éthylène, vendu sous la dénomination "SINNOWAX AO"® par la Société HENKEL | | 8 g |
| - Mélange de mono- et distéarate de glycérol non auto-émulsionnable | | 2 g |
| - Alcool cétylique | | 2 g |
| - Adipate de diisopropyle | | 10 g |
| - Huile de vaseline | | 5 g |
| - Glycérine | | 10 g |
| - Conservateurs qs. | | |
| - Eau | qsp | 100 g |

**EXEMPLE 7**

**Emulsion anti-solaire eau-dans-huile :**

- 4-tert.-butyl 4'-méthoxydibenzoylméthane — 1 g
- Silicone à greffon 2 -(3'-triméthylène-2'-hydroxy-5'-méthylphényl)benzotriazole de formule (silicone A) : — 2 g

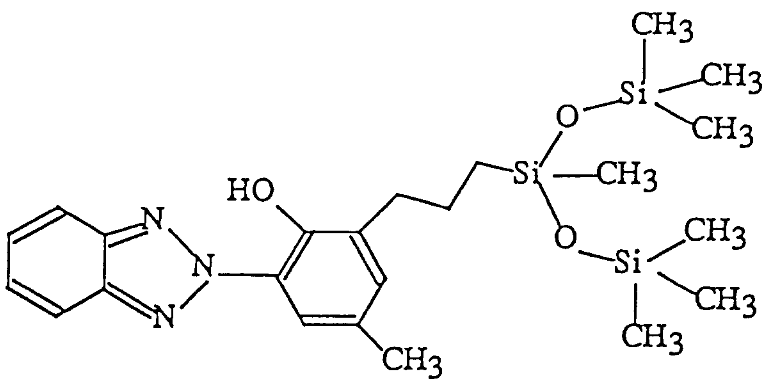

- Mélange (hydroxystéarate et isostéarate de sorbitol et de glycérol) oxypropyléné à 2 moles d'oxyde de propylène et oxyéthyléné à 3 moles d'oxyde d'éthylène, vendu sous la dénomination "ARLACEL 780"® par la Société ICI — 6 g
- Huile de vaseline — 10 g
- Triglycérides d'acides caprique et caprylique vendus sous la dénomination "MIGLYOL 812"® par la Société HULS — 5 g
- 12-hydroxystéarate de bétahydroxyoctacosanyle vendu sous la dénomination "ELFACOS C26"® par la Société AKZO — 3 g
- Mélange de cyclopentadiméthylsiloxane, cyclotétradiméthylsiloxane, cyclohexadiméthylsiloxane, vendu sous la dénomination "DC 345 FLUID"® par la Société DOW CORNING — 3 g
- Glycérine — 5 g
- Sulfate de magnésium — 0,7 g
- Conservateurs qs.
- Eau — qsp 100 g

**Modes de préparation des silicones A, B et C**

**Silicone A**

On chauffe à 40°C, sous agitation et sous azote, un mélange de 3-allyl 2-hydroxy 5-méthyl phényl benzotriazole (5 g, 18 meq), de bis(triméthylsiloxy)méthylsilane (Petrarch Systems Inc., B 2497, 4,2 g, 18 meq en SiH) et de complexe cyclovinylméthyle siloxane au platine (Petrarch, PC085, 5 µl) dans du toluène sec (8 ml) à 60-70°C. On laisse sous agitation et à 40°C jusqu'à disparition des groupements SiH (absence de bande à 2180 $cm^{-1}$ en infrarouge), soit 2 heures. On évapore le solvant. L'huile brune obtenue est chromatographiée sur colonne de silice (150 g, éluant : heptane/dichlorométhane 80:20). Après élimination des fractions de têtes, on obtient le produit attendu sous forme d'une poudre blanche (6 g, rendement = 65%).

. Spectre $^{13}C$ RMN ($CDCl_3$) : spectre conforme à la formule.
. Spectre $^{29}Si$ RMN ($CDCl_3$) : spectre conforme à la formule.
. UV (Ethanol) $\lambda_{max}$ = 303 nm, $\varepsilon_{max}$ = 16000
$\lambda_{max}$ = 342 nm, $\varepsilon_{max}$ = 15300.

| . Analyse pour $C_{23}H_{37}N_3O_3Si_3$ | | | | |
|---|---|---|---|---|
| | C | H | N | Si |
| Calculé | 56,63 | 7,65 | 8,61 | 17,27 |
| Trouvé | 57,01 | 7,71 | 8,66 | 16,94 |
| PF = 68°C. | | | | |

**Silicone B**

On ajoute, goutte à goutte, en une heure, à une solution de 3-allyl 2-hydroxy 5-méthyl phényl benzotriazole (61,6 g, 232 meq) et de complexe cyclovinylméthyle siloxane au platine (Petrarch PC085, 50 µl) dans du toluène sec (150 ml) à 80-90°C, sous azote et sous agitation, du 1,1,3,3,5,5,7,7,9,9,11,11-dodécaméthylhexasiloxane (50 g, 232 meq en SiH). On laisse sous agitation et à 80°C jusqu'à disparition des groupements SiH (absence de bande à 2180 $cm^{-1}$ en infrarouge), soit 4 heures. On évapore le solvant. L'huile visqueuse orangée obtenue (103 g) est purifiée sur colonne de silice (500 g, éluant heptane/dichlorométhane 9:1 pour éliminer les monomères, puis heptane/dichlorométhane/AcOH 90:9.8:0,2 pour donner le produit attendu sous forme d'une cire blanche (70 g, rendement = 63%).

. Spectre $^{13}C$ RMN ($CDCl_3$) : spectre conforme à la formule.
. Spectre $^{29}Si$ RMN ($CDCl_3$) : spectre conforme à la formule.
. UV (Ethanol) $\lambda_{max}$ = 302 nm, $\varepsilon_{max}$ = 30200
$\lambda_{max}$ = 340 nm, $\varepsilon_{max}$ = 27700.

| . Analyse pour $C_{44}H_{68}N_6O_7Si_6$ | | | | |
|---|---|---|---|---|
| | C | H | N | Si |
| Calculé | 54,96 | 7,13 | 8,74 | 17,52 |
| Trouvé | 54,90 | 7,12 | 8,81 | 17,11 |
| PF = 38°C. | | | | |

Silicone C

On ajoute, goutte à goutte, en 1 heure 15 minutes, à une solution de 3-méthallyl 2-hydroxy 5-méthyl phényl benzotriazole (90 g, 322 meq) et de complexe cyclovinylméthyle siloxane au platine (Petrarch PC 085, 0,3 ml) dans du toluène sec (130 ml) à 80°C, sous azote et sous agitation, du bis-(triméthylsiloxy)méthylsilane (Petrarch Systems Inc., B 2497, 75,4 g, 322 meq en SiH). On laisse sous agitation à 80°C jusqu'à disparition du benzothriazole de départ, soit 4 heures. On évapore le solvant. L'huile brune obtenue est cristallisée dans l'éthanol pour obtenir le produit attendu sous forme de poudre blanc cassé (97 g, rendement = 60%).

. Spectre $^{13}C$ RMN ($CDCl_3$) : spectre conforme à la formule.
. Spectre $^{29}Si$ RMN ($CDCl_3$) : spectre conforme à la formule.
. UV (Ethanol) $\lambda_{max}$ = 303 nm, $\varepsilon_{max}$ = 16300
$\lambda_{max}$ = 343 nm, $\varepsilon_{max}$ = 15600.

| . Analyse pour $C_{24}H_{39}N_3O_3Si_3$ | | | | |
|---|---|---|---|---|
| | C | H | N | Si |
| Calculé | 57,44 | 7,83 | 8,37 | 16,79 |
| Trouvé | 57,35 | 7,76 | 8,09 | 16,51 |
| PF = 49°C. | | | | |

## Revendications

**1.** Composition cosmétique filtrante, photostable, pour la protection de la peau et des cheveux contre les rayons ultraviolets de longueurs d'onde comprises entre 280 et 380 nm, caractérisée par le fait qu'elle comprend dans un support cosmétiquement acceptable contenant au moins une phase grasse, 0,5 à 4% en poids de 4-(tert.-butyl) 4'-méthoxy dibenzoylméthane et 0,1 à 20%, de préférence 0,5 à 15% en poids, d'un polymère filtre du type silicone benzotriazole comportant au moins une unité de formule :

$$X - \overset{\displaystyle R'_a}{\overset{|}{Si}} - O_{\frac{3-a}{2}} \qquad (I)$$

dans laquelle

R' désigne un groupe hydrocarboné saturé ou insaturé en $C_1$-$C_{30}$, un groupe hydrocarboné halogéné en $C_1$-$C_8$ ou un groupe triméthylsilyloxy, au moins 40% en nombre des radicaux R' étant des radicaux méthyle;
a = 1 ou 2;
X = - A - Y où A représente un radical divalent hydrocarboné aliphatique ou aromatique comportant au moins 2 atomes de carbone et renfermant éventuellement un ou plusieurs atomes d'oxygène;
Y représente un reste 2-(2'-hydroxyphényl)benzotriazole portant éventuellement, sur un ou les deux noyaux aromatiques, un ou plusieurs substituants alkyle en $C_1$-$C_8$, alcényle en $C_2$-$C_8$, halogène, alcoxy, carboxy, hydroxy ou amino, le rapport en poids de la silicone benzotriazole au 4-(tert.-butyl) 4'-méthoxy dibenzoylméthane étant compris entre 1 et 10.

**2.** Composition selon la revendication 1, caractérisée par le fait que la silicone benzotriazole comprend, en plus des unités de formule (I), des unités de formule :

$$R'_b\text{-Si}\,O_{\frac{4-b}{2}} \qquad (II)$$

dans laquelle R' a la même signification que dans la revendication 1, b est un nombre entier désignant 1, 2 ou 3, au moins 40% en nombre des radicaux R' étant des radicaux méthyle.

**3.** Composition cosmétique selon la revendication 1 ou 2, caractérisée par le fait qu'elle constitue une composition protectrice de l'épiderme humain ou antisolaire et se présente sous forme de lotion, lotion épaissie, gel, huile, dispersion vésiculaire, crème, lait, poudre, bâtonnet solide, mousse ou spray.

**4.** Composition cosmétique selon la revendication 1 ou 2, caractérisée par le fait qu'elle constitue une composition de maquillage des cils, des sourcils ou de la peau et se présente sous forme solide ou pâteuse, anhydre ou aqueuse, d'émulsion, de suspension, de dispersion vésiculaire.

**5.** Composition cosmétique selon la revendication 1 ou 2, utilisée pour la protection des cheveux contre les rayons ultraviolets, caractérisée par le fait qu'elle se présente sous forme de shampooing, de lotion, de gel, d'émulsion, de dispersion vésiculaire ou de laque pour cheveux.

**6.** Composition selon l'une quelconque des revendications 1 à 5, sous forme d'émulsion ou de dispersion vésiculaire de lipides amphiphiles ioniques ou non-ioniques, caractérisée par le fait qu'elle contient un filtre UV hydrosoluble choisi parmi l'acide benzène 1,4-[di(3-méthylidène 10-camphosulfonique)], l'acide 2-phénylbenzimidazole 5-sulfonique et l'acide 2-hydroxy-4-méthoxybenzophénone 5-sulfonique, salifiés ou non et présents dans la phase aqueuse.

**7.** Composition cosmétique selon l'une quelconque des revendications 1 à 6, caractérisée par le fait qu'elle comprend en outre des adjuvants cosmétiques choisis parmi les corps gras, les solvants organiques, les silicones, les épaississants, les adoucissants, les filtres solaires UV-B lipophiles, les agents anti-mousse, les agents hydratants, les parfums, les conservateurs, les tensio-actifs anioniques, cationiques, non-ioniques, amphotères ou leurs mélanges, les charges, les séquestrants, les polymères anioniques, cationiques, non-ioniques, amphotères ou leurs mélanges, les propulseurs, les agents alcalinisants ou acidifiants, les colorants et les pigments d'oxydes métalliques de granulométrie comprise entre 100 nm et 20 000 nm.

**8.** Composition selon l'une quelconque des revendications 1 à 7, caractérisée par le fait qu'elle contient en outre des nanopigments d'oxyde métallique dispersés dans la phase grasse et/ou dans la phase aqueuse.

**9.** Procédé de traitement cosmétique de la peau et des cheveux pour les protéger contre les effets des rayons UV de longueurs d'onde comprises entre 280 et 380 nm, caractérisé par le fait qu'il consiste à appliquer une quantité efficace d'une composition cosmétique filtrante telle que définie dans l'une quelconque des revendications 1 à 8.

**10.** Procédé de stabilisation du 4-(tert.-butyl) 4'-méthoxydibenzoylméthane vis-à-vis du rayonnement UV, caractérisé par le fait que l'on ajoute 0,1 à 20%, et de préférence 0,5 à 15% en poids, d'un polymère filtre du type silicone benzotriazole comportant au moins une unité de formule :

$$X\text{-}\overset{R'_a}{\underset{|}{Si}}\text{-}O_{\frac{3-a}{2}} \qquad (I)$$

dans laquelle

R' désigne un groupe hydrocarboné saturé ou insaturé en $C_1$-$C_{30}$, un groupe hydrocarboné halogéné en $C_1$-$C_8$ ou un groupe triméthylsilyloxy, au moins 40% en nombre des radicaux R' étant des radicaux méthyle;
a = 1 ou 2;
X = - A - Y où A représente un radical divalent hydrocarboné aliphatique ou aromatique comportant au moins 2 atomes de carbone et renfermant éventuellement un ou plusieurs atomes d'oxygène;
Y représente un reste 2-(2'-hydroxyphényl)benzotriazole portant éventuellement, sur un ou les deux noyaux aromatiques, un ou plusieurs substituants alkyle en $C_1$-$C_8$ alcényle en $C_2$-$C_8$, halogène, alcoxy, carboxy, hydroxy ou amino, à 0,5 à 4% en poids de 4-(tert.-butyl)-4'-méthoxydibenzoylméthane; le rapport en poids de

la silicone benzotriazole au 4-(tert.-butyl) 4'-méthoxydibenzoylméthane étant compris entre 1 et 10.

11. Procédé selon la revendication 10, caractérisé par le fait que la silicone benzotriazole comprend, en plus des unités de formule (I), des unités de formule :

$$R'_b - Si\,O_{\frac{4-b}{2}} \qquad (II)$$

dans laquelle R' a la même signification que dans la revendication 10, b est un nombre entier désignant 1, 2 ou 3, au moins 40% en nombre des radicaux R' étant des radicaux méthyle.

## Claims

1. Photostable filtering cosmetic composition for the protection of the skin and the hair against ultraviolet rays with wavelengths between 280 and 380 nm, characterized in that it comprises, in a cosmetically acceptable vehicle containing at least one fatty phase, 0.5 to 4% by weight of 4-(tert-butyl)-4'-methoxydibenzoylmethane and 0.1 to 20%, preferably 0.5 to 15% by weight, of a filtering polymer of the benzotriazole silicone type containing at least one unit of formula:

$$X - \overset{\displaystyle R'_a}{\overset{|}{Si}} - O_{\frac{3-a}{2}} \qquad (I)$$

in which

R' denotes a saturated or unsaturated $C_1$-$C_{30}$ hydrocarbon group, a $C_1$-$C_8$ halogenated hydrocarbon group or a trimethylsilyloxy group, at least 40% in numerical terms of the radicals $R^1$ being methyl radicals;
a = 1 or 2;
X = - A - Y where A represents an aliphatic or aromatic divalent hydrocarbon radical containing at least 2 carbon atoms and optionally containing one or more oxygen atoms;
Y represents a 2-(2'-hydroxyphenyl)benzotriazole residue optionally bearing, on one or both of the aromatic rings, one or more $C_1$-$C_8$ alkyl, $C_2$-$C_8$ alkenyl, halogen, alkoxy, carboxyl, hydroxyl or amino substituents, the weight ratio of the benzotriazole silicone to the 4-(tert-butyl)-4'-methoxydibenzoylmethane being between 1 and 10.

2. Composition according to Claim 1, characterized in that the benzotriazole silicone comprises, in addition to the units of formula (I), units of formula:

$$R'_b - Si\,O_{\frac{4-b}{2}} \qquad (II)$$

in which R' has the same meaning as in Claim 1 and b is an integer denoting 1, 2 or 3, at least 40% in numerical terms of the radicals R' being methyl radicals.

3. Cosmetic composition according to Claim 1 or 2, characterized in that it constitutes a composition for protecting the human epidermis or an antisun composition and is in the form of a lotion, a thickened lotion, a gel, an oil, a vesicle dispersion, a cream, a milk, a powder, a solid stick, a foam or a spray.

4. Cosmetic composition according to Claim 1 or 2, characterized in that it constitutes a make-up composition for the eyelashes, the eyebrows or the skin and is in solid or pasty, anhydrous or aqueous form, in the form of an emulsion, a suspension or a vesicle dispersion.

5. Cosmetic composition according to Claim 1 or 2, which is used for protection of the hair against ultra-violet rays, characterized in that it is in the form of a shampoo, a lotion, a gel, an emulsion, a vesicle dispersion or a lacquer for the hair.

6. Composition according to any one of Claims 1 to 5, in the form of an emulsion or vesicle dispersion of ionic or nonionic amphiphilic lipids, characterized in that it contains a water-soluble UV filter chosen from benzene-1,4-[di(3-methylidene-10-camphorsulfonic)] acid, 2-phenylbenzimidazole-5-sulphonic acid and 2-hydroxy-4-methoxybenzophenone-5-sulphonic acid, which may or may not be salified and which are present in the aqueous phase.

**7.** Cosmetic composition according to any one of Claims 1 to 6, characterized in that it additionally comprises cosmetic adjuvants chosen from fats, organic solvents, silicones, thickeners, softeners, lipophilic UV-B sunscreen agents, anti-foaming agents, hydrating agents, fragrances, preserving agents, anionic, cationic, nonionic or amphoteric surfactants or mixtures thereof, fillers, sequestering agents, anionic, cationic, nonionic or amphoteric polymers or mixtures thereof, propellants, basifying or acidifying agents, dyes and metal oxide pigments of particle size between 100 nm and 20,000 nm.

**8.** Composition according to any one of Claims 1 to 7, characterized in that it additionally contains metal oxide nanopigments dispersed in the fatty phase and/or in the aqueous phase.

**9.** Cosmetic treatment process for the skin and the hair for protecting them against the effects of UV rays with wavelengths between 280 and 380 nm, characterized in that it consists in applying an effective amount of a filtering cosmetic composition as defined in any one of Claims 1 to 8.

**10.** Process for the stabilization of 4-(tert-butyl)-4'-methoxydibenzoylmethane with regard to UV radiation, characterized in that there is added 0.1 to 20%, and preferably 0.5 to 15% by weight, of a filtering polymer of the benzotriazole silicone type containing at least one unit of formula:

$$X-\overset{R'_a}{\overset{|}{Si}}-O_{\frac{3-a}{2}} \qquad (I)$$

in which

R' denotes a saturated or unsaturated $C_1$-$C_{30}$ hydrocarbon group, a $C_1$-$C_8$ halogenated hydrocarbon group or a trimethylsilyloxy group, at least 40% in numerical terms of the radicals $R^1$ being methyl radicals;
a = 1 or 2;
X = - A - Y where A represents an aliphatic or aromatic divalent hydrocarbon radical containing at least 2 carbon atoms and optionally containing one or more oxygen atoms;
Y represents a 2-(2'-hydroxyphenyl)benzotriazole residue optionally bearing, on one or both of the aromatic rings, one or more $C_1$-$C_8$ alkyl, $C_2$-$C_8$ alkenyl, halogen, alkoxy, carboxyl, hydroxyl or amino substituents containing from 0.5 to 4% by weight of 4-(tert-butyl)-4'-methoxydibenzoylmethane; the weight ratio of the benzotriazole silicone to the 4-(tert-butyl)-4'-methoxydibenzoylmethane being between 1 and 10.

**11.** Process according to Claim 10, characterized in that the benzotriazole silicone comprises, in addition to the units of formula (I), units of formula:

$$R'_b - Si\,O_{\frac{4-b}{2}} \qquad (II)$$

in which R' has the same meaning as in Claim 10 and b is an integer denoting 1, 2 or 3, at least 40% in numerical terms of the radicals R' being methyl radicals.

## Patentansprüche

**1.** Filternde, lichtstabile kosmetische Zusammensetzung zum Schutz der Haut und der Haare vor ultravioletten Strahlen einer Wellenlänge von 280 bis 380 nm,
dadurch **gekennzeichnet**, daß
sie in einem kosmetisch geeigneten Trägermedium, das mindestens eine Fett-Phase enthält, 0,5 bis 40 Gew.% 4-(t-Butyl)-4'-methoxydibenzoylmethan und 0,1 bis 20 und vorzugsweise 0,5 bis 15 Gew.% eines polymeren Filterstoffs vom Typ einer Benzotriazol-Siliziumverbindung umfaßt, die mindestens eine Einheit der Formel aufweist:

$$X\text{-}\overset{\displaystyle R'_a}{\overset{|}{Si}}\text{-}O_{\frac{3-a}{2}} \qquad (I)$$

worin gilt:

R' bedeutet eine gesättigte oder ungesättigte $C_1$-$C_{30}$-Kohlenwasserstoff-, eine halogenierte $C_{1-8}$-Kohlenwasserstoff- oder eine Trimethylsilyloxygruppe, wobei mindetens 40% der Anzahl der Reste R' Methylreste sind;

a = 1 oder 2;

X = -A-Y,
worin A einen aliphatischen oder aromatischen zweiwertigen Kohlenwasserstoffrest darstellt, der mindestens zwei Kohlenstoffatome aufweist und gegebenenfalls ein oder mehrere Sauerstoffatome enthält;

Y stellt einen 2-(2'-Hydroxyphenyl)benzotriazol-Rest dar, der gegebenenfalls, auf einem oder den beiden aromatischen Kernen, einen oder mehrere $C_{1-8}$-Alkyl-, $C_{2-8}$-Alkenyl-, Halogen-, Alkoxy-, Carboxy-, Hydroxy- oder Amino-Substituenten aufweist, wobei das Gewichtsverhältnis der Benzotriazol-Siliziumverbindung zum 4-(t-Butyl)-4'-methoxydibenzoylmethan 1 bis 10 beträgt.

**2.** Zusammensetzung gemäß Anspruch 1,
dadurch **gekennzeichnet**, daß
die Benzotriazol-Siliziumverbindung, zusätzlich zu den Einheiten der Formel (I), Einheiten der Formel aufweist:

$$R'_b - Si\,O_{\frac{4-b}{2}} \qquad (II)$$

worin R' dieselbe Bedeutung wie in Anspruch 1 hat und b eine ganze Zahl von 1´, 2 oder 3 ist, wobei mindestens 40% der Anzahl der Reste R' Methylreste sind.

**3.** Kosmetische Zusammensetzung gemäß Anspruch 1 oder 2,
dadurch **gekennzeichnet**, daß
sie eine Schutzmittelzusammensetzung für die menschliche Epidermis oder eine Sonnenschutzmittelzusammensetzung darstellt und in Form einer Lotion, verdickten Lotion, eines Gels, Öls, einer bläschenartigen Dispersion, Creme, Milch, eines Pulvers bzw. Puders, Feststoffstäbchens, Schaums oder Spray vorliegt.

**4.** Kosmetische Zusammensetzung gemäß Anspruch 1 oder 2,
dadurch **gekennzeichnet**, daß
sie eine Zusammensetzung zum Schminken der Wimpern, Augenbrauen oder der Haut darstellt und in fester oder pasteuser, wasserfreier oder wässriger Form einer Emulsion, Suspension oder bläschenartigen Dispension vorliegt.

**5.** Kosmetische Zusammensetzung gemäß Anspruch 1 oder 2 zur Verwendung zum Schutz der Haare vor ultravioletten Strahlen,
dadurch **gekennzeichnet**, daß
sie in Form eines Shampoo, einer Lotion, eines Gels, einer Emulsion, bläschenartigen Dispersion oder eines Lacks für Haare vorliegt.

**6.** Zusammensetzung gemäß einem der Anprüche 1 bis 5 in Form einer Emulsion oder bläschenartigen Dispersion aus amphiphilen ionischen oder nicht-ionischen Lipiden,
dadurch **gekennzeichnet**, daß
sie einen wasserlöslichen UV-Filterstoff enthält, der aus 1,4-(Di(3-methyliden-10-kamphosulfonsäure))benzol, 2-Phenylbenzimidazol-5-sulfonsäure und aus 2-Hydroxy-4-methoxybenzophenon-5-sulfonsäure ausgewählt ist, welche in die Salzform überführt sind oder nicht und in der wässrigen Phase vorliegen.

**7.** Kosmetische Zusammensetzung gemaß einem der Ansprüche 1 bis 6,

dadurch **gekennzeichnet**, daß
sie ausserdem kosmetische Hilfsstoffe enthält, ausgewählt aus Fettkörpern, organischen Lösungsmitteln, Siliconen, Verdickungsmitteln, weichmachenden Mitteln, lipophilen Sonnenfilterstoffen für UV-B, Antischaummitteln, hydratisierenden Mitteln, Parfüm-Produkten, Konservierungsstoffen, anionischen, kationischen, nicht-ioonischen oder amphoteren oberflächenaktiven Mitteln oder deren Mischungen, Beaufschlagungsmitteln, Sequestriermitteln, anionischen, kationischen, nicht-ionische oder amphoteren Polymeren oder deren Mischungen, Treibmitteln, alkalisch oder sauer stellenden Mitteln, Farbstoffen und aus Pigmenten aus Metalloxiden einer Korngröße von 100 bis 20 000 nm.

**8.** Zusammensetzung gemäß einem der Ansprüche 1 bis 7,
dadurch **gekennzeichnet**, daß
sie ausserdem Nanopigmente aus Metalloxid enthält, die in der fetthaltigen oder wässrigen Phase dispergiert sind.

**9.** Verfahren zur kosmetischen Behandlung der Haut und der Haare zu deren Schutz vor Ein- bzw. Auswirkungen von UV-Strahlen einer Wellenlänge von 280 bis 380 nm,
dadurch **gekennzeichnet**, daß
es darauf beruht, daß man eine wirksame Menge einer in jedem der Ansprüche 1 bis 8 definierten kosmetischen Filterzusammensetzung zur Anwendung bringt.

**10.** Verfahren zum Stabilisieren von 4-(t-Butyl)-4'-methoxydibenzoylmethan vor UV-Strahlung,
dadurch **gekennzeichnet**, daß
man 0,1 bis 20 und vorzugsweise 0,5 bis 15 Gew.% eines polymeren Filterstoffs vom Typ einer Benzotriazol-Siliziumverbindung, die mindestens eine Einheit der Formel aufweist:

$$X-\overset{R'_a}{\overset{|}{Si}}-O_{\frac{3-a}{2}} \qquad (I)$$

worin gilt:

R' bedeutet eine gesättigte oder ungesättigte $C_{1-30}$-Kohlenwasserstoff-, eine halogenierte $C_{1-8}$-Kohlenwasserstoff- oder eine Trimethylsilyloxygruppe, wobei mindetens 40% der Anzahl der Reste R' Methylreste sind;

a = 1 oder 2;

X = -A-Y,
worin A einen aliphatischen oder aromatischen zweiwertigen Kohlenwasserstoffrest darstellt, der mindestens zwei Kohlenstoffatome aufweist und gegebenenfalls ein oder mehrere Sauerstoffatome enthält;

Y stellt einen 2-(2'-Hydroxyphenyl)benzotriazol-Rest dar, der gegebenenfalls, auf einem oder den beiden aromatischen Kernen, einen oder mehrere $C_{1-8}$-Alkyl-, $C_{2-8}$-Alkenyl-, Halogen-, Alkoxy-, Carboxy-, Hydroxy- oder Amino-Substituenten aufweist, wobei das Gewichtsverhältnis der Benzotriazol-Siliziumverbindung zum 4-(t-Butyl)-4'-methoxydibenzoylmethan 1 bis 10 beträgt.

**11.** Verfahren gemäß Anspruch 10,
dadurch **gekennzeichnet**, daß
die Benzotriazol-Siliziumverbindung, zusätzlich zu den Einheiten der Formel (I), Einheiten der Formel aufweist:

$$R'_b - Si\,O_{\frac{4-b}{2}} \qquad (II)$$

worin R' dieselbe Bedeutung wie in Anspruch 10 hat und b eine ganze Zahl von 1, 2 oder 3 ist, wobei mindestens 40% der Anzahl der Reste R' Methylreste sind.